(19) Europäisches Patentamt
European Patent Office
Office européen des brevets

(11) EP 4 620 934 A1

(12) EUROPEAN PATENT APPLICATION
published in accordance with Art. 153(4) EPC

(43) Date of publication:
**24.09.2025 Bulletin 2025/39**

(21) Application number: **23910411.0**

(22) Date of filing: **22.12.2023**

(51) International Patent Classification (IPC):
***C04B 26/04*** *(2006.01)* ***C04B 38/00*** *(2006.01)*
***C04B 41/63*** *(2006.01)* ***A61K 6/891*** *(2020.01)*
***C04B 111/80*** *(2006.01)*

(52) Cooperative Patent Classification (CPC):
**A61K 6/891; C04B 26/04; C04B 38/00;**
**C04B 41/63;** C04B 2111/80

(86) International application number:
**PCT/CN2023/141105**

(87) International publication number:
**WO 2024/140485 (04.07.2024 Gazette 2024/27)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.12.2022 CN 202211687527**

(71) Applicant: **Aidite (Qinhuangdao) Technology Co., Ltd.**
**Qinhuangdao Hebei 066004 (CN)**

(72) Inventors:
- **ZHANG, Jiaxin**
  **Qinhuangdao, Hebei 066004 (CN)**
- **WEI, Jiaxin**
  **Qinhuangdao, Hebei 066004 (CN)**
- **QIAO, Chunmei**
  **Qinhuangdao, Hebei 066004 (CN)**
- **LIU, Qianqian**
  **Qinhuangdao, Hebei 066004 (CN)**
- **MENG, Zhe**
  **Qinhuangdao, Hebei 066004 (CN)**

(74) Representative: **Cicconetti, Andrea**
**Accapi S.r.l.**
**Via de' Griffoni, 10/A**
**40123 Bologna (IT)**

# (54) RESIN INFILTRATED CERAMIC AND PREPARATION METHOD THEREFOR

(57) The present invention relates to a resin infiltrated ceramic and a preparation method therefor. The resin infiltrated ceramic comprises a ceramic blank and resin provided inside the ceramic blank and on the surface of same. The ceramic blank comprises polymethyl methacrylate modified sodium aluminum silicate. Raw materials for preparation of the resin comprise an acrylate-type resin monomer and polyhedral oligomeric silsesquioxane containing a methacrylate functional group. The resin infiltrated ceramic disclosed by the present invention has excellent mechanical properties, has fracture toughness, flexural strength and hardness which are similar to that of human teeth, and has the characteristic of low shrinkage, thus facilitating CAD/CAM cutting. Therefore, the resin infiltrated ceramic can be used as a dental material for crown and bridge prosthodontics.



Processed by Luminess, 75001 PARIS (FR)

## Description

The present invention claims the priority to the Chinese patent application No. CN202211687527.7 filed on December 27, 2022 and entitled "RESIN INFILTRATED CERAMIC AND PREPARATION METHOD THEREFOR", the contents of which are incorporated herein by reference in entirety.

## TECHNICAL FIELD

The present invention relates to the technical field of resin infiltrated ceramics, in particular to a resin infiltrated ceramic and a preparation method therefor.

## BACKGROUND ART

Resin infiltrated ceramic is a novel composite transparent ceramic material with an interpenetrating network structure formed by infiltrating resin into a porous ceramic scaffold. It represents the forefront of advancements in digital crown and bridge restoration diagnostics, as well as material innovation and research in dentistry.

CN106007802A discloses a resin infiltrated ceramic composite material and a preparation method therefor. This resin infiltrated ceramic adopts yttria-stabilized tetragonal zirconia as the porous ceramic blank, with a mixed resin consisting of methacrylate-type resin and benzoyl peroxide thermal curing agent. The prepared resin infiltrated ceramic exhibits excellent mechanical properties, but the material differs significantly from natural human teeth, lacking biomimetic characteristics and making it unsuitable for clinical use. For example, the performance of the elastic modulus of the prepared material is much higher than that of human dentin. When used clinically by patients, the restorative material can wear to human natural teeth and cause injury.

CN107903557A discloses a machinable resin infiltrated glass-ceramic material for dentistry and the preparation method therefor. The resin infiltrated ceramic material adopts silanized barium glass-ceramic powder as the porous ceramic blank, with acrylic acrylate resin monomer and benzoyl peroxide as the resin to be infiltrated. The prepared resin infiltrated glass-ceramic material for dental restoration has mechanical properties close to those of human teeth, but its elastic modulus is lower than that of natural teeth.

CN108578250A discloses a resin infiltrated silicate composite material and the preparation method therefor. The common bisphenol A glycidyl methacrylate and tetraethylene glycol dimethacrylate, with benzoyl peroxide as the curing agent are selected as the resin to be infiltrated. The resin is infiltrated into the porous ceramic block via vacuum capillary action and then cured to form a silicate/resin dual-network structure. However, the bending strength and hardness of this resin infiltrated silicate composite material are relatively low.

In the prior art, research on resin infiltrated ceramics remains limited. The ceramic bodies are primarily based on zirconia ceramics, alumina ceramics, or glass ceramics, to form porous ceramic frameworks. The resulting resin infiltrated ceramics exhibit significant differences in mechanical properties compared to natural human teeth. These differences can lead to wear on natural human teeth or negatively affect the transmission of occlusal stress between the restorative material and natural teeth.

Therefore, it is necessary to develop a resin infiltrated ceramic material with an elastic modulus closely matching that of human teeth.

## SUMMARY

To address the deficiencies of the prior art, the objective of the present invention is to provide a resin infiltrated ceramic and a preparation method therefor. The resin infiltrated ceramic exhibits excellent mechanical properties, including fracture toughness, flexural strength, and hardness comparable to human teeth, along with low shrinkage characteristics. It is easily machinable via CAD/CAM and can serve as a dental material for crown and bridge prosthodontics.

In order to achieve the above objective of the present invention, the following technical solution is adopted.

In the first aspect, a resin infiltrated ceramic is provided in the present invention, wherein the resin infiltrated ceramic includes a ceramic blank and a resin disposed within and on the surface of the ceramic blank.

The ceramic blank includes polymethyl methacrylate-modified sodium aluminosilicate.

The raw materials for preparing the resin include an acrylate-type resin monomer and a polyhedral oligomeric silsesquioxane containing methacrylate functional group.

In the present invention, polymethyl methacrylate-modified sodium aluminosilicate serves as the main material of a ceramic blank, used in combination with the resin system. Within the resin system, the polyhedral oligomeric silsesquioxane containing methacrylate functional group has a structure similar to that of an acrylate-type resin, so that the reduction of the polymerization shrinkage rate of the resin infiltrated ceramic can be realized, and the good compatibility

and dispersibility between the incorporated component and the acrylic resin can be ensured. Additionally, the excellent interfacial compatibility between the polymethyl methacrylate-modified sodium aluminosilicate and the acrylate resin ensures the good compatibility and dispersibility between them and the resin, thereby endowing the resin infiltrated ceramic with superior fracture toughness, flexural strength, and hardness.

In the present invention, no specific restrictions are imposed on the acrylate-type resin monomers used for preparing the resin, as long as the practical requirements are met. In the present invention, the acrylate-type resin monomer can be monofunctional (methyl) acrylates or polyfunctional (methyl) acrylates. Illustratively, the acrylate-type resin monomer include any one of: methyl (methyl)acrylate, ethyl (methyl)acrylate, 2-hydroxyethyl (methyl)acrylate, butyl (methyl)acrylate, benzyl (methyl)acrylate, tetrahydrofurfuryl (methyl)acrylate or isobornyl (methyl)acrylate, glycol mono(4-cumylphenyl) ether methacrylate (CMP-1E), 2-(2-biphenyloxy)ethyl methacrylate, bisphenol A dimethacrylate, Bis-GMA (the addition product of methacrylic acid and bisphenol A diglycidyl ether), UDMA (the addition product of 2-hydroxyethyl methacrylate and 2,2,4-trimethyl hexamethylene-1,6-diisocyanate), diethylene glycol dimethacrylate, triethylene glycol dimethacrylate, tetraethylene glycol dimethacrylate, trimethylolpropane trimethacrylate, pentaerythritol tetramethacrylate, glycerol dimethacrylate, glycerol trimethacrylate, 1,4-butanediol dimethacrylate, 1,10-decanediol dimethacrylate (D3MA), bis(methacryloyloxymethyl)tricyclo-[5.2.1.02,6]decane (DCP), polyethylene glycol dimethacrylate or polypropylene glycol dimethacrylate, polyethylene glycol 200 dimethacrylate, polyethylene glycol 400 dimethacrylate, and 1,12-dodecanediol dimethacrylate, or any mixture thereof.

Preferably, the mass percentage of the ceramic blank is 70%-80%, such as 72%, 74%, 76%, or 78%, based on 100% of the total mass of the resin infiltrated ceramic.

Preferably, the mass percentage of the resin is 20%-30%, such as 22%, 24%, 26%, or 28%, based on 100% of the total mass of the resin infiltrated ceramic.

Preferably, the porosity of the ceramic blank is 10%-55%, such as 20%, 30%, 40% or 50%.

Preferably, the raw materials for preparing the ceramic blank include a sodium aluminosilicate, a solvent, a silane coupling agent, a methyl methacrylate monomer, and an initiator.

Preferably, the particle size of the sodium aluminosilicate is 0.4μm-3.2μm, such as 0.5μm, 1μm, 1.5μm, 2μm, 2.5μm, or 3μm.

Preferably, the solvent includes anhydrous ethanol.

Preferably, the initiator includes azobisisobutyronitrile.

Preferably, based on 100% of the total mass of the raw materials for preparing the ceramic blank, the raw materials include the following components in mass percentage:

| | |
|---|---|
| sodium aluminosilicate | 20%-40% |
| solvent | 30%-60% |
| silane coupling agent | 10%-25% |
| methyl methacrylate monomer | 15%-25% |
| initiator | 0.02%-0.1%. |

In the present invention, the mass percentage of the sodium aluminosilicate is 20%-40%, for example 25%, 30% or 35%.

In the present invention, the mass percentage of the solvent is preferably 30%-60%, for example 35%, 40%, 45%, 50% or 55%.

In the present invention, the mass percentage of the silane coupling agent is preferably 10%-25%, for example 12%, 14%, 16%, 18%, 20%, 22% or 24%.

In the present invention, the mass percentage of the methyl methacrylate monomer is preferably 15%-25%, for example 16%, 18%, 20%, 22% or 24%.

In the present invention, the mass percentage of the initiator is preferably 0.02%-0.1%, for example 0.04%, 0.06% or 0.08%.

Preferably, the chemical formula of the polyhedral oligomeric silsesquioxane containing methacrylate functional group is $(RSiO_{3/2})n$,

where the n is 6, 8, 10, 12 or 14, and at least one R is selected from methacrylate functional group.

Polyhedral oligomeric silsesquioxane (POSS) is a class of organic-inorganic hybrid monomers with a particle size of approximately 1.5nm, and the molecular general formula is $(RSiO_{3/2})n$, where n is typically 6, 8, or 10. POSS exhibits properties intermediate between silicon dioxide ($SiO_2$) and silicone resins $(R_2SiO)n$. It has a polyhedral inorganic framework core with SiO nanostructures, and its periphery is surrounded by organic groups. The organic groups of functionalized polyhedral oligomeric silsesquioxane possess reactivity and can generate good compatibility or chemical

bond action with a plurality of polymers to form the nano-scale inorganic-organic hybrid composite material.

Preferably, the structural formula of the polyhedral oligomeric silsesquioxane containing methacrylate functional group (POSS-MA) is shown in formula I:

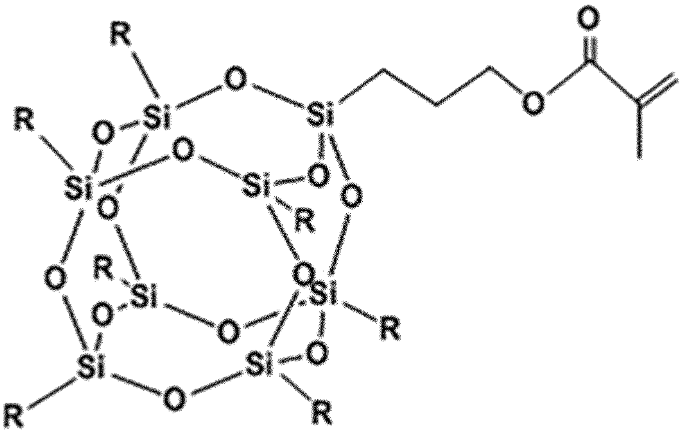

**Formula I,**

where each R is independently selected from hydrogen, methacrylate functional group, or other functional groups containing the unsaturated bond, with the methacrylate functional group being further preferred.

The present invention preferably employs a POSS derivative with 8 R groups as methacrylate functional groups, namely polyhedral oligomeric silsesquioxane-methacrylate (POSS-MA). POSS-MA is combined with an acrylic resin, the two exhibit good compatibility so that the curing reaction can be carried out simultaneously. When being added into the dental resin material, the inorganic scaffold structure of them can maintain the spatial structure during polymerization, thus effectively reducing volumetric shrinkage of the material, so that the interface crack caused by the polymerization volumetric shrinkage between the resin after impregnation and curing and the ceramic framework is effectively avoided, and the resin infiltrated ceramic material is endowed with excellent mechanical property.

All acrylate-based POSS involved in the present invention can be synthesized or purchased commercially.

Preferably, the raw materials for preparing the resin further include a thermal initiator.

Preferably, the thermal initiator includes any one or a combination of at least two of dicumyl peroxide, tert-butyl peroxide and benzoyl peroxide, wherein typical but nonlimiting combinations include a combination of dicumyl peroxide and tert-butyl peroxide, a combination of tert-butyl peroxide and benzoyl peroxide, a combination of dicumyl peroxide, tert-butyl peroxide and benzoyl peroxide.

Preferably, the tert-butyl peroxide includes tert-butyl peroxyacetate and/or tert-butyl peroxybenzoate.

Preferably, based on 100% of the total mass of the raw materials for preparing the resin, the raw materials for preparing include the following components in mass percentage:

| | |
|---|---|
| acrylate-type resin monomer | 70%-95% |
| polyhedral oligomeric silsesquioxane containing methacrylate functional group | 0.003%-18% |
| thermal initiator | 0.35%-2%. |

In the present invention, the mass percentage of the acrylate-type resin monomer is preferably 70%-95%, for example 75%, 80% or 85%.

In the present invention, the mass percentage of the polyhedral oligomeric silsesquioxane containing methacrylate functional group is preferably 0.003%-18%, for example 0.5%, 1%, 2%, 4%, 6%, 8%, 10%, 12%, 14%, 16% or 18%.

In the present invention, the mass percentage of the thermal initiator is preferably 0.35%-2%, for example 0.5%, 1% or 1.5%.

In the second aspect, a method for preparing the resin infiltrated ceramic according to the first aspect is provided in the present invention, wherein the method includes the following step: infiltrating a resin into a ceramic blank to obtain the resin infiltrated ceramic.

Preferably, the method for preparing the resin infiltrated ceramic includes the following steps:

(1) mixing an acrylate-type resin monomer, a thermal initiator and a polyhedral oligomeric silsesquioxane containing methacrylate functional group, and reacting to obtain the resin;

mixing the sodium aluminosilicate, the solvent, the silane coupling agent, the methyl methacrylate monomer and the initiator, carrying out in-situ polymerization, and pressing to obtain the ceramic blank, and
(2) impregnating the ceramic blank in resin, infiltrating and curing, so as to obtain the resin infiltrated ceramic.

Preferably, in step (1), the in-situ polymerization includes:

(a) mixing and dispersing the sodium aluminosilicate, the solvent, the silane coupling agent, and the methyl methacrylate monomer, heating for reaction and then post treating, and
(b) mixing the raw material obtained by the post treating in the step (a), the methyl methacrylate monomer and the initiator for reaction to complete the in-situ polymerization.

In the present invention, the sodium aluminosilicate with a lower cost is selected as the porous ceramic scaffold for the resin infiltrated ceramic. The aim is mainly to graft polymethyl methacrylate (PMMA) on the surface of the sodium aluminosilicate for interface modification by an in-situ polymerization method, thereby obtaining the PMMA-coated sodium aluminosilicate nano composite material, which is used as the ceramic framework material for the resin infiltrated ceramic. On one hand, the uniform dispersibility of the sodium aluminosilicate in the resin infiltrated ceramic can be improved, making the formation of interpenetrating network channels easy; on the other hand, the interface compatibility between the sodium aluminosilicate and the acrylic resin can be improved. Since the combination between the sodium aluminosilicate and PMMA is completely based on a stable chemical bond, which is equivalent to the PMMA molecular chain "growing" on the sodium aluminosilicate nanoparticles, the difference between an inorganic phase and an organic phase is eliminated. The mechanical properties of the resin infiltrated ceramic thus prepared are remarkably improved, thereby providing a resin infiltrated ceramic material with excellent mechanical properties (the fracture toughness, flexural strength, and hardness are comparable to those of human teeth) and low shrinkage, making the resin infiltrated ceramic is easily machinable via CAD/CAM, so that it can be used as a dental material for crown and bridge prosthodontics.
Preferably, in step (a), the method of mixing and dispersing includes stirring and sonication.
Preferably, the temperature of the heating for reaction is 70°C-90°C, for example, 75°C, 80°C or 85°C.
Preferably, the duration of the heating for reaction is 1h-5h, for example 1.5h, 2h, 2.5h, 3h, 3.5h, 4h or 4.5h.
Preferably, the post treating includes washing and drying.
Preferably, in step (b), the mixing includes first mixing the raw material obtained by the post treating in step (a) with a methyl methacrylate monomer and then mixing a resultant with an initiator.
Preferably, the reaction includes a first reaction, a second reaction and a third reaction.
Preferably, the temperature of the first reaction is 70°C-80°C (for example, 72°C, 74°C, 76°C, or 78°C), until the system becomes viscous.
Preferably, the duration of the first reaction is 3h-5h, for example 3.5h, 4.5h or 5h.
Preferably, the temperature of the second reaction is 40°C-60°C, for example 45°C, 50°C or 55°C.
Preferably, the duration of the second reaction is 10h-30h, for example 15h, 20h or 25h.
Preferably, the temperature of the third reaction is 90°C-110°C, for example 95°C, 100°C or 105°C.
Preferably, the duration of the third reaction is 1h-5h, for example 1.5h, 2h, 2.5h, 3h, 3.5h, 4h or 4.5h.
Preferably, the pressing pressure is 1MPa-10MPa, for example 2MPa, 4MPa, 6MPa or 8MPa.
Preferably, the pressing duration is 1min-5min, for example 2min, 3min or 4min.
Preferably, in step (2), the infiltrating is carried out under vacuum.
Preferably, the infiltrating duration is 3h-4 h, for example 3.2h, 3.4h, 3.6h or 3.8h.
Preferably, the curing temperature is 120°C-160°C, for example 130°C, 140°C or 150°C.
Preferably, the curing pressure is 200MPa-300MPa, for example 220MPa, 240MPa, 260MPa or 280MPa.
Preferably, the curing duration is 60min-90min, for example 65min, 70min, 75min, 80min or 85min.
As a preferred technical solution, the method for preparing the resin infiltrated ceramic includes the following steps:

(1) mixing an acrylate-type resin monomer, a thermal initiator and a polyhedral oligomeric silsesquioxane containing methacrylate functional group, and reacting to obtain the resin;

mixing and dispersing a sodium aluminosilicate, a solvent, a silane coupling agent and a methyl methacrylate monomer under stirring and sonication, and then reacting for 1h-5h at the temperature of 70°C-90°C, washing and drying, sequentially mixing the obtained raw materials with the methyl methacrylate monomer and an initiator, reacting at the temperature of 70°C-80°C until the system becomes viscous, continuously reacting for 10h-30h at the temperature of 40°C-60°C, and finally reacting for 1h-5h at the temperature of 90°C-110°C, so as to obtain the raw material of ceramic blank, and
impregnating the raw material of ceramic blank in a dispersion liquid, and pressing for 1min-5min under

1MPa-10MPa, so as to obtain the ceramic blank;

(2) impregnating the ceramic blank in resin, infiltrating for 3h-4h under vacuum, and curing for 60min-90min under the condition of the temperature being 120°C-160°C and the pressure being 200MPa-300MPa, so as to obtain the resin infiltrated ceramic.

Compared with the prior art, the present invention has the following beneficial effects:

(1) the resin infiltrated ceramic disclosed by the present invention exhibits excellent mechanical properties, including fracture toughness, flexural strength, and hardness comparable to those of human teeth, along with low shrinkage characteristics. It is easily machinable via CAD/CAM and can serve as a dental material for crown and bridge prosthodontics, and
(2) the resin infiltrated ceramic disclosed by the present invention has the elastic modulus between 20.5 ±1.4GPa-24.2±1.4GPa, the bending strength between 211.8±5.4MPa-291.8±8.1MPa, the Vickers hardness between 2.2±0.2GPa-3.3±0.2GPa, and the shrinkage rate within 1.87%.

## DETAILED DESCRIPTION OF EMBODIMENTS

In the following, the technical solutions of the present invention will be further explained by specific embodiments. It should be apparent to those skilled in the art that the described examples are merely to aid in the understanding of the present invention and should not be taken as a specific limitation of the present invention.

### Example 1

In the present example, a resin infiltrated ceramic was provided, wherein the resin infiltrated ceramic included a ceramic blank and a resin disposed within and on a surface of the ceramic blank.
The ceramic blank included the polymethyl methacrylate-modified sodium aluminosilicate.
The raw materials for preparing the resin included an acrylate-type resin monomer and a polyhedral oligomeric silsesquioxane containing methacrylate functional group.
In the present example, the structural formula of the polyhedral oligomeric silsesquioxane containing methacrylate functional group (POSS-MA) was shown in formula I:

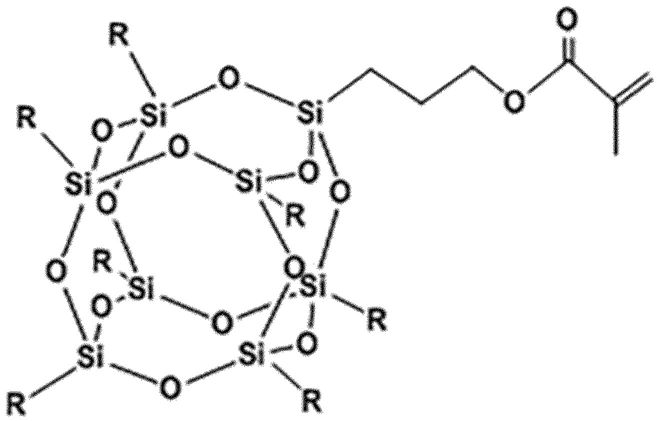

**Formula I,**

where R was a methacrylate functional group, in particular to methacryloyloxypropyl, wherein the structural formula was as following:

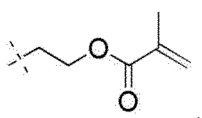

The resin infiltrated ceramic was obtained by the following preparation method, wherein the preparation method included the following steps:

(1) Preparation of the resin

0.5% benzoyl peroxide and 5% POSS-MA were introduced into triethylene glycol dimethacrylate, and the mixture was stirred at 150rev/min for 1h at room temperature. Subsequently, bisphenol A glycidyl methacrylate was weighed at a 5:5 ratio and added to the above system, followed by stirring for 2h until thoroughly mixed. The mixture was then removed and set aside for later use.

(2) Preparation of sodium aluminosilicate ceramic powder

1000mL of anhydrous ethanol, 30g of KH-570, and 30g of sodium aluminosilicate powder were added to a 3000mL three-necked flask. The mixture was ultrasonically dispersed and stirred in an ultrasonic cleaner for 15min, then heated under reflux in an 80°C water bath for 3h. After the reaction, centrifugation was performed, and the precipitate was washed three times with anhydrous ethanol and distilled water, respectively. The resultant was vacuum-dried and finally ground finely with a mortar, so as to obtain the modified sodium aluminosilicate, which was set aside for later use.

300mL of MMA was added to a 3000mL three-necked flask, followed by the addition of 600g of the modified sodium aluminosilicate. After ultrasonic oscillation and dispersion for 30 min, AIBN was added, and pre-polymerization was carried out under stirring in a 75°C water bath. When the system became viscous after 3h of reaction, it was quickly poured into a 50mL test tube and further polymerized in a 50°C water bath for 20h. Finally, the temperature was raised to 100°C, and the reaction continued for 3h. After cooling and demolding, the polymethyl methacrylate-modified sodium aluminosilicate was obtained, which served as the sodium aluminosilicate ceramic powder.

(3) Preparation of the ceramic blank

100g of the prepared sodium aluminosilicate ceramic powder was weighed and uniformly impregnated with 20mL of a 3% polyvinyl alcohol aqueous solution. After drying in an 80°C oven, the powder was transferred to a steel mold and pressed at 5MPa for 3min, so as to form a ceramic blank with 25% porosity.

(4) Preparation of resin infiltrated ceramic

The ceramic blank prepared in the above steps was completely immersed in the resin system and placed in a vacuum device integrally, ensuring a vacuum level of - 0.1MPa. The infiltration was maintained under vacuum state for 3h, so as to ensure the body was fully impregnated by the resin system.

The sodium aluminosilicate ceramic blank, fully impregnated with resin, was placed in an isostatic press with constant temperature and cured under high temperature and pressure at 130°C and 300MPa for 60min, so as to obtain the translucent sodium aluminosilicate-based resin infiltrated ceramic block, namely the resin infiltrated ceramic.

**Example 2**

In the present example, a resin infiltrated ceramic was provided, wherein the resin infiltrated ceramic included a ceramic blank and a resin disposed within and on a surface of the ceramic blank.

The ceramic blank included the polymethyl methacrylate-modified sodium aluminosilicate.

The raw materials for preparing the resin included an acrylate-type resin monomer and a polyhedral oligomeric silsesquioxane containing methacrylate functional group.

The preparation method of the present example differed from Example 1 only in step (2): the preparation of sodium aluminosilicate ceramic powder, while the remaining steps were identical to those in Example 1. The specific step (2) in the present example was as follows:

1000mL of anhydrous ethanol, 30g of KH-570, and 30g of sodium aluminosilicate powder were added to a 3000mL three-necked flask. The mixture was ultrasonically dispersed and stirred in an ultrasonic cleaner for 15min, then heated under reflux in an 80°C water bath for 3h. After the reaction, centrifugation was performed, and the precipitate was washed three times with anhydrous ethanol and distilled water, respectively. The resultant was vacuum-dried and finally ground finely with a mortar, so as to obtain the modified sodium aluminosilicate, which was set aside for later use.

300mL of MMA was added to a 3000mL three-necked flask, followed by the addition of 300g of the modified sodium aluminosilicate. After ultrasonic oscillation and dispersion for 30 min, AIBN was added, and pre-polymerization was carried out under stirring in a 75°C water bath. The reactant was observed, and when it began to become viscous, it was quickly poured into a 50mL test tube and further polymerized in a 50°C water bath for 20h. Finally, the temperature was raised to 100°C, and the reaction continued for 3h. After cooling and demolding, the polymethyl methacrylate-modified sodium aluminosilicate was obtained, which served as the sodium aluminosilicate ceramic powder.

**Example 3**

In the present example, a resin infiltrated ceramic was provided, wherein the resin infiltrated ceramic included a ceramic blank and a resin disposed within and on a surface of the ceramic blank.
The ceramic blank included the polymethyl methacrylate-modified sodium aluminosilicate.
The raw materials for preparing the resin included an acrylate-type resin monomer and a polyhedral oligomeric silsesquioxane containing methacrylate functional group.
The preparation method of the present example differed from Example 1 only in step (2): the preparation of sodium aluminosilicate ceramic powder, while the remaining steps were identical to those in Example 1. The specific step (2) in the present example was as follows:
1000mL of anhydrous ethanol, 30g of KH-570, and 30g of sodium aluminosilicate powder were added to a 3000mL three-necked flask. The mixture was ultrasonically dispersed and stirred in an ultrasonic cleaner for 15min, then heated under reflux in an 80°C water bath for 3h. After the reaction, centrifugation was performed, and the precipitate was washed three times with anhydrous ethanol and distilled water, respectively. The resultant was vacuum-dried and finally ground finely with a mortar for later use.
300mL of MMA was added to a 3000mL three-necked flask, followed by the addition of 100g of the modified sodium aluminosilicate. After ultrasonic oscillation and dispersion for 30 min, AIBN was added, and pre-polymerization was carried out under stirring in a 75°C water bath. The reactant was observed, and when it began to become viscous, it was quickly poured into a 50mL test tube and further polymerized in a 50°C water bath for 20h. Finally, the temperature was raised to 100°C, and the reaction continued for 3h. After cooling and demolding, the polymethyl methacrylate-modified sodium aluminosilicate was obtained, which served as the sodium aluminosilicate ceramic powder.

**Example 4**

In the present example, a resin infiltrated ceramic was provided, wherein the resin infiltrated ceramic included a ceramic blank and a resin disposed within and on a surface of the ceramic blank.
The ceramic blank included the polymethyl methacrylate-modified sodium aluminosilicate.
The raw materials for preparing the resin included an acrylate-type resin monomer and a polyhedral oligomeric silsesquioxane containing methacrylate functional group.
In the present example, the polyhedral oligomeric silsesquioxane containing methacrylate functional group was the same as in Example 1.
The resin infiltrated ceramic was obtained by the following preparation method, wherein the preparation method included the following steps:

(1) Preparation of the resin

1.5% benzoyl peroxide and 12% POSS-MA were introduced into triethylene glycol dimethacrylate, and the mixture was stirred at 150rev/min for 1h at room temperature. Subsequently, UDMA (the addition product of 2-hydroxyethyl methacrylate and 2,2,4-trimethyl hexamethylene-1,6-diisocyanate) was weighed at a 3:7 ratio and added to the above system, followed by stirring for 2h until thoroughly mixed. The mixture was then removed and set aside for later use.

(2) Preparation of sodium aluminosilicate ceramic powder

1000mL of anhydrous ethanol, 30g of KH-570, and 120g of sodium aluminosilicate powder were added to a 3000mL three-necked flask. The mixture was ultrasonically dispersed and stirred in an ultrasonic cleaner for 20min, then heated under reflux in an 80°C water bath for 3h. After the reaction, centrifugation was performed, and the precipitate was washed three times with anhydrous ethanol and distilled water, respectively. The resultant was vacuum-dried and finally ground finely with a mortar, so as to obtain the modified sodium aluminosilicate, which was set aside for later use.
400mL of MMA was added to a 3000mL three-necked flask, followed by the addition of 80g of the modified sodium aluminosilicate. After ultrasonic oscillation and dispersion for 30 min, AIBN was added, and pre-polymerization was carried out under stirring in a 72°C water bath. When the system became viscous after 5h of reaction, it was quickly poured into a 1000mL beaker and further polymerized in a 60°C water bath for 15h. Finally, the temperature was raised to 105°C, and the reaction continued for 4h. After cooling and demolding, the polymethyl methacrylate-modified sodium aluminosilicate was obtained, which served as the sodium aluminosilicate ceramic powder.

(3) Preparation of the ceramic blank

100g of the prepared sodium aluminosilicate ceramic powder was weighed and uniformly impregnated with

20mL of a 3% polyvinyl alcohol aqueous solution. After drying in an 80°C oven, the powder was transferred to a steel mold and pressed at 6MPa for 2min, so as to form a ceramic blank with 22% porosity.

(4) Preparation of resin infiltrated ceramic

The ceramic blank prepared in the above steps was completely immersed in the resin system and placed in a vacuum device integrally, ensuring a vacuum level of - 0.1MPa. The infiltration was maintained under vacuum state for 3.5h, so as to ensure the body was fully impregnated by the resin system.

The sodium aluminosilicate ceramic blank, fully impregnated with resin, was placed in an isostatic press with constant temperature and cured under high temperature and pressure at 140°C and 250MPa for 70min, so as to obtain the translucent sodium aluminosilicate-based resin infiltrated ceramic block, namely the resin infiltrated ceramic.

**Example 5**

In the present example, a resin infiltrated ceramic was provided, wherein the resin infiltrated ceramic included a ceramic blank and a resin disposed within and on a surface of the ceramic blank.

The ceramic blank included the polymethyl methacrylate-modified sodium aluminosilicate.

The raw materials for preparing the resin included an acrylate-type resin monomer and a polyhedral oligomeric silsesquioxane containing methacrylate functional group.

In the present example, the polyhedral oligomeric silsesquioxane containing methacrylate functional group was the same as in Example 1.

The resin infiltrated ceramic was obtained by the following preparation method, wherein the preparation method included the following steps:

(1) Preparation of the resin

1.5% benzoyl peroxide and 8% POSS-MA were introduced into 1,10-decanediol dimethacrylate, and the mixture was stirred at 150rev/min for 1h at room temperature. Subsequently, bisphenol A glycidyl methacrylate was weighed at a 4:6 ratio and added to the above system, followed by stirring for 2h until thoroughly mixed. The mixture was then removed and set aside for later use.

(2) Preparation of sodium aluminosilicate ceramic powder

1000mL of anhydrous ethanol, 30g of KH-570, and 120g of sodium aluminosilicate powder were added to a 3000mL three-necked flask. The mixture was ultrasonically dispersed and stirred in an ultrasonic cleaner for 20min, then heated under reflux in an 80°C water bath for 3h. After the reaction, centrifugation was performed, and the precipitate was washed three times with anhydrous ethanol and distilled water, respectively. The resultant was vacuum-dried and finally ground finely with a mortar, so as to obtain the modified sodium aluminosilicate, which was set aside for later use.

400mL of MMA was added to a 3000mL three-necked flask, followed by the addition of 80g of the modified sodium aluminosilicate. After ultrasonic oscillation and dispersion for 30 min, AIBN was added, and pre-polymerization was carried out under stirring in a 78°C water bath. When the system became viscous after 3.5h of reaction, it was quickly poured into a 1000mL beaker and further polymerized in a 60°C water bath for 15h. Finally, the temperature was raised to 110°C, and the reaction continued for 3h. After cooling and demolding, the polymethyl methacrylate-modified sodium aluminosilicate was obtained, which served as the sodium aluminosilicate ceramic powder.

(3) Preparation of the ceramic blank

100g of the prepared sodium aluminosilicate ceramic powder was weighed and uniformly impregnated with 25mL of a 3% polyvinyl alcohol aqueous solution. After drying in an 80°C oven, the powder was transferred to a steel mold and pressed at 6MPa for 2min, so as to form a ceramic blank with 28% porosity.

(4) Preparation of resin infiltrated ceramic

The ceramic blank prepared in the above steps was completely immersed in the resin system and placed in a vacuum device integrally, ensuring a vacuum level of - 0.1MPa. The infiltration was maintained under vacuum state for 4h, so as to ensure the body was fully impregnated by the resin system.

The sodium aluminosilicate ceramic blank, fully impregnated with resin, was placed in an isostatic press with constant temperature and cured under high temperature and pressure at 140°C and 220MPa for 90min, so as to obtain the

translucent sodium aluminosilicate-based resin infiltrated ceramic block, namely the resin infiltrated ceramic.

**Example 6**

The difference between the present example and Example 1 was that in the polyhedral oligomeric silsesquioxane containing methacrylate functional group, R was a vinyl group, while all other conditions remained the same as in Example 1.

**Comparative Example 1**

The difference between the present comparative example and Example 1 was that the ceramic blank was an unmodified sodium aluminosilicate, namely, the step (2) was omitted during preparation, and in step (3) the sodium aluminosilicate was used. All other conditions remained the same as in Example 1.

**Comparative Example 2**

The difference between the present comparative example and Example 1 was that in the raw material for the preparation of the resin, the polyhedral oligomeric silsesquioxane containing methacrylate functional group was replaced with the polyhedral oligomeric silsesquioxane without containing methacrylate functional group, wherein the structural formula shown below, R was a vinyl group. All other conditions remained the same as in Example 1.

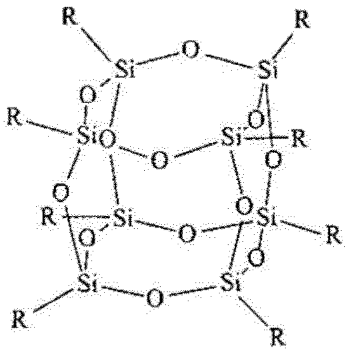

**Comparative Example 3**

The difference between the present comparative example and Example 1 was that in the raw material for the preparation of the resin, the polyhedral oligomeric silsesquioxane containing methacrylate functional group was replaced with the polyhedral oligomeric silsesquioxane without containing methacrylate functional group, wherein the structural formula shown below, R was an aldehyde group. All other conditions remained the same as in Example 1.

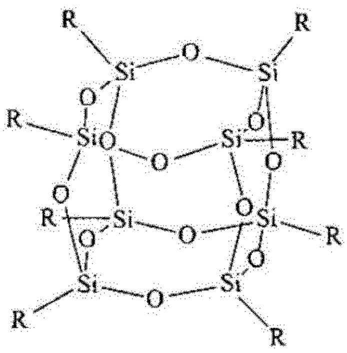

**Comparative Example 4**

The difference between the present comparative example and Example 1 was that the sodium aluminosilicate was replaced with an equal mass of zirconium oxide, while all other conditions remained the same as in Example 1.

**Test of the performance**

The resin infiltrated ceramics prepared in Examples 1-6 and Comparative Examples 1-4, along with human teeth as a blank control, were subjected to the following tests:

(1) Elastic modulus and bending strength: the resin infiltrated ceramics were cut into test specimens measuring 1.2mm × 4.0mm × 18mm, then wet-polished with 2000-grit sandpaper to polish the surface. A three-point bending test was performed using a tensile testing machine under the conditions of a fulcrum spacing of 12mm and a crosshead speed of 1.0mm/min. The average values from ten specimens were used for evaluation.
(2) Vickers hardness: the test surfaces of resin infiltrated ceramics were polished with 2000-grit sandpaper under water to ensure the smooth surfaces of the sample. Testing with Vickers hardness tester: the test force value was selected as "2". The average value was obtained by measuring three different positions of the resin infiltrated ceramic.
(3) Shrinkage: the shrinkage of resin infiltrated ceramics was characterized by volumetric shrinkage rate. That was, the volumetric shrinkage rate was calculated by measuring the density of the same resin infiltrated ceramic block before and after curing.

$$\text{Total Volumetric Shrinkage} = \frac{\text{Density of Cured Sample - Density of Uncured Sample}}{\text{Density of Cured Sample}} \times 100\%$$

The corresponding test results are summarized in Table 1.

Table 1 Performance test results of resin infiltrated ceramics prepared in Examples and Comparative Examples, along with the blank control material

| | Elastic Modulus (GPa) | Bending Strength (MPa) | Vickers Hardness (GPa) | Shrinkage (%) |
|---|---|---|---|---|
| Example 1 | 21.7±1.7 | 221.8±10.1 | 2.4±0.2 | 1.65 |
| Example 2 | 22.5±1.2 | 251.6±5.1 | 2.7±0.2 | 1.52 |
| Example 3 | 24.2±1.4 | 291.8±8.1 | 3.3±0.2 | 1.47 |
| Example 4 | 23.5±0.8 | 264.7±3.4 | 3.0±0.2 | 1.51 |
| Example 5 | 22.4±1.3 | 258.2±6.2 | 2.8±0.2 | 1.55 |
| Example 6 | 20.5±1.4 | 211.8±5.4 | 2.2±0.2 | 1.87 |
| Comparative Example 1 | 13.2±6.1 | 179±4.7 | 2.1±1.2 | 4.23 |
| Comparative Example 2 | 17.2±2.3 | 199.8±6.7 | 2.0±0.6 | 3.23 |
| Comparative Example 3 | 16.5±3.2 | 202.4±5.7 | 1.9±0.2 | 3.19 |
| Comparative Example 4 | 19.2±1.2 | 188.8±7.2 | 2.4±1.2 | 2.64 |
| Blank Comparative Example | 20~25 | 180~300 | 2~4 | / |

Analysis of that data in table 1 show that the resin infiltrated ceramic provided in the present invention has the elastic modulus between 20.5±1.4GPa-24.2±1.4GPa, the bending strength between 211.8±5.4MPa-291.8±8.1MPa, the Vickers hardness between 2.2±0.2GPa-3.3±0.2GPa, and the shrinkage rate within 1.87%. The resin infiltrated ceramic provided in the present invention exhibits excellent mechanical properties, including fracture toughness, flexural strength, and hardness comparable to those of human teeth, along with low shrinkage characteristics. It is easily machinable via CAD/CAM and can serve as a dental material for crown and bridge prosthodontics.
Analysis of Comparative Examples 1-4 and Example 1 shows that the performance of the products provided in Comparative Examples 1-4 is inferior to that of Example 1, proving the superior performance of the resin infiltrated ceramics of the present invention.
Analysis of Example 6 and Example 1 shows that the performance of the product provided in Example 6 is inferior to that of Example 1, proving that when R groups in the polyhedral oligomeric silsesquioxane containing methacrylate functional groups are all methacrylate functional groups, the performance of resin infiltrated ceramics is further improved.
The present invention has been described in detail through the above examples. However, the present invention is not limited to the above detailed methods, that is, it is not meant that the present invention must rely on the above detailed

methods to be practiced. Those skilled in the art should understand that any modifications to the present invention, equivalent substitutions of raw materials, additions of auxiliary components, or selection of specific methods all fall within the protection scope and disclosure range of the present invention.

## Claims

1. A resin infiltrated ceramic, **characterized in that** the resin infiltrated ceramic comprises a ceramic blank and a resin disposed within and on a surface of the ceramic blank;

   the ceramic blank comprises a polymethyl methacrylate-modified sodium aluminosilicate, and
   a raw material for preparing the resin comprises an acrylate-type resin monomer and a polyhedral oligomeric silsesquioxane containing methacrylate functional group.

2. The resin infiltrated ceramic according to claim 1, wherein a mass percentage of the ceramic blank is 70%-80%, based on 100% of a total mass of the resin infiltrated ceramic, and
   a mass percentage of the resin is 20%-30%, based on 100% of the total mass of the resin infiltrated ceramic.

3. The resin infiltrated ceramic according to claims 1 or 2, wherein a porosity of the ceramic blank is 10%-55%.

4. The resin infiltrated ceramic according to any one of claims 1-3, wherein a raw material for preparing the ceramic blank comprises a sodium aluminosilicate, a solvent, a silane coupling agent, a methyl methacrylate monomer, and an initiator;
   based on 100% of a total mass of the raw material for preparing the ceramic blank, the raw material comprises following components:

| | |
|---|---|
| sodium aluminosilicate | 20%-40% |
| solvent | 30%-60% |
| silane coupling agent | 10%-25% |
| methyl methacrylate monomer | 15%-25% |
| initiator | 0.02%-0.1%. |

5. The resin infiltrated ceramic according to claim 4, wherein a particle size of the sodium aluminosilicate is 0.4μm-3.2μm;

   the solvent comprises an anhydrous ethanol, and
   the initiator comprises an azobisisobutyronitrile.

6. The resin infiltrated ceramic according to any one of claims 1-3, wherein a chemical formula of the polyhedral oligomeric silsesquioxane containing methacrylate functional group is $(RSiO_{3/2})n$,

   where the n is 6, 8, 10, 12 or 14, and at least one R is selected from a methacrylate functional group;
   the raw material for preparing the resin further comprises a thermal initiator;
   the thermal initiator comprises any one or a combination of at least two of a dicumyl peroxide, a tert-butyl peroxide and a benzoyl peroxide, and
   the tert-butyl peroxide comprises a tert-butyl peroxyacetate and/or a tert-butyl peroxybenzoate.

7. The resin infiltrated ceramic according to claim 6, wherein a structural formula of the polyhedral oligomeric silsesquioxane containing methacrylate functional group is shown in formula I:

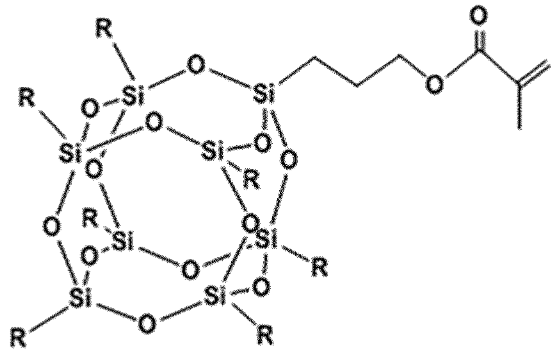

**Formula I,**

where each R is independently selected from a hydrogen, a methacrylate functional group, or other functional groups containing an unsaturated bond.

**8.** The resin infiltrated ceramic according to claim 6, wherein based on 100% of the total mass of the raw material for preparing the resin, the raw material comprises following components in mass percentage:

| | |
|---|---|
| the acrylate-type resin monomer | 70%-95% |
| the polyhedral oligomeric silsesquioxane containing methacrylate functional group | 0.003%-18% |
| the thermal initiator | 0.35%-2%. |

**9.** The resin infiltrated ceramic according to claim 1, 7, or 8, wherein the acrylate-type resin monomer comprises a monofunctional (methyl) acrylate or a polyfunctional (methyl) acrylate.

**10.** The resin infiltrated ceramic according to claim 9, wherein the acrylate-type resin monomer comprises any one of: methyl (methyl)acrylate, ethyl (methyl)acrylate, 2-hydroxyethyl (methyl)acrylate, butyl (methyl)acrylate, benzyl (methyl)acrylate, tetrahydrofurfuryl (methyl)acrylate or isobornyl (methyl)acrylate, glycol mono(4-cumylphenyl) ether methacrylate, 2-(2-biphenyloxy)ethyl methacrylate, bisphenol A dimethacrylate, an addition product of methacrylic acid and bisphenol A diglycidyl ether, an addition product of 2-hydroxyethyl methacrylate and 2,2,4-trimethyl hexamethylene-1,6-diisocyanate, diethylene glycol dimethacrylate, triethylene glycol dimethacrylate, tetraethylene glycol dimethacrylate, trimethylolpropane trimethacrylate, pentaerythritol tetramethacrylate, glycerol dimethacrylate, glycerol trimethacrylate, 1,4-butanediol dimethacrylate, 1,10-decanediol dimethacrylate, bis(methacryloyloxymethyl)tricyclo-[5.2.1.02,6]decane, polyethylene glycol dimethacrylate or polypropylene glycol dimethacrylate, polyethylene glycol 200 dimethacrylate, polyethylene glycol 400 dimethacrylate, and 1,12-dodecanediol dimethacrylate, or any mixture thereof.

**11.** A preparation method for the resin infiltrated ceramic according to any one of claims 1-10, **characterized in that** the method comprises a following step: infiltrating the resin into the ceramic blank to obtain the resin infiltrated ceramic.

**12.** The preparation method according to claim 11, wherein the preparation method comprises following steps:

(1) mixing an acrylate-type resin monomer, a thermal initiator and a polyhedral oligomeric silsesquioxane containing methacrylate functional group, and reacting to obtain the resin;
mixing a sodium aluminosilicate, a solvent, a silane coupling agent, a methyl methacrylate monomer and an initiator, carrying out an in-situ polymerization, and pressing to obtain the ceramic blank, and
(2) impregnating the ceramic blank in the resin, infiltrating and curing to obtain the resin infiltrated ceramic.

**13.** The preparation method according to claim 11, wherein in the step (1), the in-situ polymerization comprises:

(a) mixing and dispersing the sodium aluminosilicate, the solvent, the silane coupling agent, the methyl methacrylate monomer, heating for reaction and post treating, and
(b) mixing a raw material obtained by the post treating in the step (a), the methyl methacrylate monomer and the initiator for reaction to complete the in-situ polymerization.

**14.** The preparation method according to claim 13, wherein in the step (a), a method of mixing and dispersing comprises a stirring and a sonication;

a temperature of the heating for reaction is 70°C-90°C;
a duration of the heating for reaction is 1h-5h, and
the post treating comprises a washing and a drying.

**15.** The preparation method according to claim 13, wherein in the step (b), the mixing comprises first mixing the raw material obtained by the post treating in the step (a) with the methyl methacrylate monomer and then mixing a resultant with the initiator.

**16.** The preparation method according to claim 13, wherein in the step (b), the reaction comprises a first reaction, a second reaction and a third reaction;

a temperature of the first reaction is 70°C-80°C until the system becomes viscous;
a duration of the first reaction is 3h-5h;
a temperature of the second reaction is 40°C-60°C;
a duration of the second reaction is 10h-30h;
a temperature of the third reaction is 90°C-110°C, and
a duration of the third reaction is 1h-5h.

**17.** The preparation method according to claim 12, wherein a pressing pressure is 1MPa-10MPa, and
a pressing duration is 1min-5min.

**18.** The preparation method according to claim 12, wherein in the step (2), the infiltrating is carried out under a vacuum;

an infiltrating duration is 3h-4h;
a curing temperature is 120°C-160°C;
a curing pressure is 200MPa-300MPa, and
a curing duration is 60min-90min.

**19.** The preparation method according to any one of claims 11-18, wherein the preparation method comprises following steps:

(1) mixing an acrylate-type resin monomer, a thermal initiator and a polyhedral oligomeric silsesquioxane containing methacrylate functional group, and reacting to obtain the resin;

mixing and dispersing a sodium aluminosilicate, a solvent, a silane coupling agent and a methyl methacrylate monomer under stirring and sonication, and then reacting for 1h-5h at a temperature of 70°C-90°C, washing and drying, sequentially mixing the obtained raw material with a methyl methacrylate monomer and an initiator, reacting at a temperature of 70°C-80°C until the system becomes viscous, continuously reacting for 10h-30h at a temperature of 40°C-60°C, and finally reacting for 1h-5h at a temperature of 90°C-110°C, so as to obtain a raw material of a ceramic blank, and
impregnating the raw material of the ceramic blank in a dispersion liquid, and pressing for 1min-5min under 1MPa-10MPa, so as to obtain the ceramic blank;

(2) impregnating the ceramic blank in the resin, infiltrating for 3h-4h under vacuum, and curing for 60min-90min under a condition of a temperature being 120°C-160°C and a pressure being 200MPa-300MPa, so as to obtain the resin infiltrated ceramic.

## INTERNATIONAL SEARCH REPORT

International application No.
**PCT/CN2023/141105**

**A. CLASSIFICATION OF SUBJECT MATTER**

C04B26/04(2006.01)i; C04B38/00(2006.01)i; C04B41/63(2006.01)i; A61K6/891(2020.01)i; C04B111/80(2006.01)n

According to International Patent Classification (IPC) or to both national classification and IPC

**B. FIELDS SEARCHED**

Minimum documentation searched (classification system followed by classification symbols)

IPC: C04B A61K

Documentation searched other than minimum documentation to the extent that such documents are included in the fields searched

Electronic data base consulted during the international search (name of data base and, where practicable, search terms used)

CNTXT; CNKI; WPABS; VEN; WOTXT; EPTXT; USTXT; ISI_WEB OF SCIENCE: 爱迪特, 张佳新, 魏嘉欣, 乔春梅, 刘乾乾, 孟哲, 渗透, 陶瓷, 硅铝酸钠, 硅铝酸钾, 甲基丙烯酸酯, infiltration, ceramic+, sodium aluminosilicate, potassium aluminosilicate, methacrylate

**C. DOCUMENTS CONSIDERED TO BE RELEVANT**

| Category* | Citation of document, with indication, where appropriate, of the relevant passages | Relevant to claim No. |
|---|---|---|
| PX | CN 116023066 A (AIDITE (QINGHUANGDAO) TECHNOLOGY CO., LTD.) 28 April 2023 (2023-04-28)<br>claims 1-10 | 1-19 |
| Y | JP H1043209 A (G C DENTARU PROD:KK) 17 February 1998 (1998-02-17)<br>description, paragraphs 8-37 | 1-19 |
| Y | CN 115105416 A (AIDITE (QINGHUANGDAO) TECHNOLOGY CO., LTD.) 27 September 2022 (2022-09-27)<br>description, paragraphs 7-84 | 1-19 |
| A | CN 106007802 A (TSINGHUA UNIVERSITY) 12 October 2016 (2016-10-12)<br>claims 1-10 | 1-19 |
| A | CN 107903557 A (BEIJING OYA BORUI SCIENCE & TECHNOLOGY CO., LTD. et al.) 13 April 2018 (2018-04-13)<br>claims 1-10 | 1-19 |
| A | CN 108524291 A (TSINGHUA UNIVERSITY) 14 September 2018 (2018-09-14)<br>claims 1-11 | 1-19 |

☐ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **19 March 2024** | **20 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**
**Information on patent family members**

International application No.
**PCT/CN2023/141105**

| Patent document cited in search report | | | Publication date (day/month/year) | Patent family member(s) | | | Publication date (day/month/year) |
|---|---|---|---|---|---|---|---|
| CN | 116023066 | A | 28 April 2023 | None | | | |
| JP | H1043209 | A | 17 February 1998 | JP | 4636514 | B2 | 23 February 2011 |
| | | | | EP | 0803241 | A2 | 29 October 1997 |
| | | | | US | 5869548 | A | 09 February 1999 |
| | | | | EP | 0803241 | A3 | 22 December 1999 |
| | | | | EP | 0803241 | B1 | 14 June 2006 |
| | | | | DE | 69736088 | D1 | 27 July 2006 |
| | | | | DE | 69736088 | T2 | 11 January 2007 |
| CN | 115105416 | A | 27 September 2022 | CN | 115105416 | B | 21 November 2023 |
| CN | 106007802 | A | 12 October 2016 | CN | 106007802 | B | 17 September 2019 |
| CN | 107903557 | A | 13 April 2018 | CN | 107903557 | B | 20 October 2020 |
| CN | 108524291 | A | 14 September 2018 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202211687527
- CN 106007802 A
- CN 107903557 A
- CN 108578250 A